# EUROPEAN PATENT SPECIFICATION

(11) **EP 3 727 489 B1**
(45) Date of publication and mention of the grant of the patent: **09.09.2026**
(21) Application number: 18890488.2
(22) Date of filing: 23.12.2018
(51) Int. Cl.: D01D 5/00, D01F 6/82, A61L 27/18, A61L 27/56, A61L 27/58, C08G 69/44

(54) **NOVEL ELECTROSPUN SYNTHETIC DENTAL BARRIER MEMBRANES FOR GUIDED TISSUE REGENERATION AND GUIDED BONE REGENERATION APPLICATIONS**
NEUE ELEKTROGESPONNENE KÜNSTLICHE ZAHNSPERRMEMBRANEN FÜR GESTEUERTE GEWEBEREGENERATIONS- UND GESTEUERTE KNOCHENREGENERATIONSANWENDUNGEN
NOUVELLES MEMBRANES DE BARRIÈRE DENTAIRE SYNTHÉTIQUES ÉLECTROFILÉES POUR RÉGÉNÉRATION TISSULAIRE GUIDÉE ET APPLICATIONS DE RÉGÉNÉRATION OSSEUSE GUIDÉE

(30) Priority: 23.12.2017 US 201762610155 P
(43) Date of publication of application: 28.10.2020
(73) Proprietor: Matregenix, Inc., Irvine, CA 92617 (US); Soliman, Sherif, Framingham, Massachusetts 01701 (US)
(72) Inventor: KAMEL, Mohamad Ayad, Wayland, Massachusetts 01778 (US); SOLIMAN, Sherif, Irvine, CA 92617 (US)
(74) Representative: Schlich
(86) International application number: PCT/US2018/067427
(87) International publication number: WO 2019/126819

(56) References cited:
- CN-A- 1 878 514
- US-A1- 2009 022 811
- US-A1- 2009 246 259
- US-A1- 2016 250 382
- US-B2- 8 974 815
- MARY BETH WADE ET AL: "Influence of Sterilization Technologies on Electrospun Poly(ester urea)s for Soft Tissue Repair", BIOMACROMOLECULES, vol. 17, no. 10, 10 October 2016 (2016-10-10), US, pages 3363 - 3374, XP055767261, ISSN: 1525-7797, DOI: 10.1021/acs.biomac.6b01158
- ANGÉLICA DÍAZ ET AL: "New poly(ester urea) derived from l-leucine: Electrospun scaffolds loaded with antibacterial drugs and enzymes", MATERIALS SCIENCE AND ENGINEERING C, vol. 46, 1 January 2015 (2015-01-01), CH, pages 450 - 462, XP055577540, ISSN: 0928-4931, DOI: 10.1016/j.msec.2014.10.055
- OSTROWSKA ZOFI A ET AL: "Correspondence to: Zofi a Ostrowska", ACCEPTED NEUROENDOCRINOLOGY LETTERS, vol. 23, no. 23, 1 January 2002 (2002-01-01), pages 417 - 425, XP055829116
- GALLO MARTA ET AL: "Resorption of calcium phosphate materials: Considerations on thein vitroevaluation", JOURNAL OF THE EUROPEAN CERAMIC SOCIETY, ELSEVIER SCIENCE PUBLISHERS, BARKING, ESSEX, GB, vol. 38, no. 3, 10 July 2017 (2017-07-10), pages 899 - 914, XP085292813, ISSN: 0955-2219, DOI: 10.1016/J.JEURCERAMSOC.2017.07.004
- YU ET AL.: "Phenylalanine-Based Poly(ester urea): Synthesis, Characterization, and in vitro Degradation", MACROMOLECULES, vol. 47, 16 December 2013 (2013-12-16), pages 121 - 129, XP055144945, Retrieved from the Internet <URL:https://pubs.acs.org/doi/abs/10.1021/ma401752b> [retrieved on 20190226], DOI: doi:10.1021/ma401752b

## Description

### BACKGROUND

### Field of the Invention

The present disclosure relates to synthetic barrier membranes for guided tissue regeneration (GTR) and guided bone regeneration (GBR) in dental applications.

### Description of the Related Art

Periodontal disease is a major public health issue. Nearly 50% of adults in the U.S. have some form of periodontitis. *See* Rodriguez, I.A., et al. "Barrier Membranes for Dental Applications: A Review and Sweet Advancement in Membrane Developments," Mouth Teeth, 2018, 2(1), 1-9, doi: 10.15761/MTJ.1000108. Regeneration of lost periodontal tissues requires the use of barrier dental membrane devices to prevent soft-tissue invasion into the defect and guide the bone regeneration process. *See* Dimitriou, R., et al. "The Role of Barrier Membranes for Guided Bone Regeneration and Restoration of Large Bone Defects: Current Experimental and Clinical Evidence," BMC Med. 2012, 10(81), doi: 10.1186/1741-7015-10-81. Biocompatibility, space-making ability, the ability to achieve tissue integration, and clinical manageability are criteria that must be considered in the design of materials used for regenerative procedures. *See* Scantlebury, T.. "1982-1992: A Decade of Technology Development for Guided Tissue Regeneration," J. Periodontol. 1993, 64, 11-29.

A number of barrier membranes have been used to achieve the desired reconstruction, but each type of barrier membrane that has been used falls short of satisfying all of the aforementioned criteria. The current available membranes may be classified as non-resorbable or resorbable. Non-resorbable membranes have the disadvantage of requiring a second surgery to remove the membrane, which often carries a risk of infection and patient discomfort. The application of non-resorbable membranes requires a high level of surgical skill to trim and shape the membrane for use, and the use of non-resorbable membranes has exhibited an unacceptable degree of failure. *See* Bottino, M.C., et al. "Recent Advances in the Development of GTR/GBR Membranes for Periodontal Regeneration-A Materials Perspective," Dent. Mater. 2012, 28(7), 703-21. The majority of currently available resorbable membranes are composed of collagen, on account of its excellent biocompatibility. While the use of a collagen membrane eliminates the need for a second surgery, collagen membranes typically absorb at a higher rate than ideally needed to maintain the needed physical space for regeneration and to achieve surgical goals. *See* Rodella, L.F., et al. "Biomaterials in Maxillofacial Surgery: Membranes and Grafts," Int. J. Biomed. Sci. 2011, 7(2), 81-88. In addition, animal derivative materials such as collagen present problems of rejection, variability, and insufficient mechanical strength. *See* Dori, F., et al. "Effect of Platelet-Rich Plasma on the Healing of Intra-Bony Defects Treated with a Natural Bone Mineral and a Collagen Membrane," J. Clin. Periodontol. 2007, 34(3), 254-61. More recently, synthetic membranes have been used to address the limitations associated with collagen membranes. Currently available synthetic membranes are based on lactides, glycolides, and lactones. The resorption mechanism for the available synthetic membrane materials is typically limited to enzymatic and hydrolytic processes, which results in local acidification of tissue, thereby causing inflammation and bone erosion. *See* Azevedo, H.S., et al. "Understanding the Enzymatic Degradation of Biodegradable Polymers and Strategies to Control Their Degradation Rate," In Biodegradable Systems in Tissue Engineering and Regenerative Medicine, Reis, et al. Eds., 2004, 177-201. The currently available synthetic membranes degrade via a bulk erosion mechanism, which makes it very difficult to predict the degradation rate of the membranes. *Id.* Moreover, studies show that the use of currently available synthetic membranes leads to epithelial downgrowth, gingival recession, device exposure, and pronounced soft tissue inflammation. *See* Laurell, L., et al. "Gingival Response to GTR Therapy in Monkeys Using Two Bioresorbable Devices (Abstract 824)," J. Dent. Res. 1993, 72, 206. As a result, these membranes are not widely used in dental clinics. It is thus clear that the "ideal" membrane for use in periodontal regenerative therapy has yet to be developed. The generation of a dental membrane that overcomes all of the aforementioned structural, mechanical, and bio-functional limitations would have a significant positive impact on the field of guided tissue regeneration and guided bone regeneration.

Recently developed methods of scaffolding for tissue engineering offer promise in this regard. Electrospinning is a process that uses an electric field to generate continuous fibers on a micrometer or nanometer scale. Electrospinning has been shown as a promising method for the fabrication of tissue engineering scaffolds, as the resultant structures mimic the topology of the native extracellular matrix. *See, e.g.,* Li, W.J., et al. "Electrospun Nanofibrous Structure: A Novel Scaffold for Tissue Engineering," J. Biomed. Mater. Res. 2002, 60(4), 613-21; Pham, Q.P., et al. "Electrospinning of Polymeric Nanofibers for Tissue Engineering Applications: A Review," Tissue Eng. 2006, 12(5), 1197-211; Murugan, R., et al. "Nano-Featured Scaffolds for Tissue Engineering: A Review of Spinning Methodologies," Tissue Eng. 2006, 12(3), 435-47. Electrospinning enables direct control of the microstructure of a scaffold, including characteristics such as the fiber diameter, orientation, pore size, and porosity. Electrospinning has been extensively investigated as a technique for producing tunable scaffolds for various tissue engineering applications. It is believed that electrospun fibers are effective as tissue regenerative scaffolds because of their ability to mimic the fibrous extra-cellular matrix (ECM) of human tissues. However, the use of electrospun scaffolds also presents challenges for many tissue engineering applications. For example, it is difficult to promote cellular penetration into the depth of an electrospun structure, despite the porosity and flexibility afforded by the electrospinning process. The small pore size provided by the non-woven fibrous dense structure leads to a preference for cells to proliferate and migrate across the surface of the scaffold rather than inside it.

Synthetic biomaterials have gained substantial interest in various tissue engineering applications, on account of the physical and biological properties of such materials. *See, e.g.,* Yoshimoto, H., et al. "A Biodegradable Nanofiber Scaffold by Electrospinning and Its Potential for Bone Tissue Engineering," Biomaterials, 2003, 24, 2077; Albertsson, A.-C., et al. "Recent Developments in Ring Opening Polymerization of Lactones for Biomedical Applications," Biomacromolecules, 2003, 4, 1466; Hutmacher, D.W., et al. "Mechanical Properties and Cell Cultural Response of Polycaprolactone Scaffolds Designed and Fabricated via Fused Deposition Modeling," J. Biomed. Mater. Res. 2001, 55, 203; Li, W.-J., et al. "Biological Response of Chondrocytes Cultured in Three-Dimensional Nanofibrous Poly(ε-caprolactone) Scaffolds," J. Biomed. Mater. Res. A, 2003, 67A, 1105; Lin, W.-J., et al. "A Novel Fabrication of Poly(ε-caprolactone) Microspheres from Blends of Poly(ε-caprolactone) and Poly(ethylene glycol)s," Polymer, 1999, 40, 1731; Zong, X., et al. "Structure and Morphology Changes During In Vitro Degradation of Electrospun Poly(glycolide-co-lactide) Nanofiber Membrane," Biomacromolecules, 2003, 4, 416; Kim, C.H., et al. "An Improved Hydrophilicity via Electrospinning for Enhanced Cell Attachment and Proliferation," J. Biomed. Mater. Res. B Appl. Biomater. 2006, 78B, 283. Polymeric synthetic biomaterials have been shown to degrade mainly by simple hydrolysis of the polymer backbone bonds into acidic monomers, which are subsequently removed from the body by normal metabolic pathways. However, most polymeric synthetic biomaterials have a hydrophobic surface, thus limiting the applications in which they may be used. In addition, most polymeric synthetic biomaterials are also known for their slow degradation rate due to the presence of a hydrophobic surface that thus retards hydrolysis.

Electrospinning of biomaterials such as polycaprolactone, polylactic-co-glycolic acid, and chitosan has been used to generate guided tissue regeneration (GTR) and guided bone regeneration (GBR) barrier membranes. *See, e.g.,* Xue, J., et al. "Electrospun Microfiber Membranes Embedded with Drug-Loaded Clay Nanotubes for Sustained Antimicrobial Protection," ACS Nano, 2015, 9(2), 1600-12; Carter, P., et al. "Facile Fabrication of Aloe Vera Containing PCL Nanofibers for Barrier Membrane Application," J. Biomater. Sci. Polym. Ed. 2016, 27(7), 692-708, doi: 10.1080/09205063.2016.1152857; Yang, F., et al. "Development of an Electrospun Nano-Apatite/PCL Composite Membrane for GTR/GBR Application," Acta Biomater. 2009, 5, 3295-3304, doi: 10.1016/j.actbio.2009.05.023; Jia, J., et al. "Preparation and Characterization of Soluble Eggshell Membrane Protein/PLGA Electrospun Nanofibers for Guided Tissue Regeneration Membrane," J. Nanomater. 2012, doi: 10.1155/2012/282736; Qasim, S.B., et al. "Potential of Electrospun Chitosan Fibers as a Surface Layer in Functionally Graded GTR Membrane for Periodontal Regeneration," Dent. Mater. 2017, 33(1), 71-83. The use of copolymers, composites, and blends of the biomaterials such as polycaprolactone, polylactic-co-glycolic acid, and chitosan results in GTR and GBR barrier membranes with properties that are better suited for the desired applications. *See, e.g.,* Liao, S., et al. "A Three-Layered Nano-Carbonated Hydroxyapatite/Collagen/PLGA Composite Membrane for Guided Tissue Regeneration," Biomaterials, 2005, 26(36), 7564-71; Bottino, M.C., et al. "A Novel Spatially Designed and Functionally Graded Electrospun Membrane for Periodontal Regeneration," Acta Biomater. 2011, 7(1), 216-24. These electrospun GTR and GBR barrier membranes are nonetheless limited by the properties of the biomaterials used.

Wade et al., "Influence of Sterilization Technologies on Electrospun Poly(Ester Urea)s for Soft Tissue Repair," Biomacromolecules, 2016, 17, 3363-74 describes the electrospinning of degradable poly(ester urea)s into nanofiber sheets and the assessment for their potential use in soft tissue repair. Diaz et al., "New Poly(Ester Urea) Derived from L-Leucine: Electrospun Scaffolds Loaded with Antibacterial Drugs and Enzymes," Mater. Sci. Eng. C, 2015, 46, 450-62 describes electrospun scaffolds from an amino acid containing poly(ester urea) for use in tissue engineering applications. U.S. Patent No. 8,974,815 B2 to Chu, et al*.* describes electrospun biodegradable poly(ester-amide) fabrics which are said to be suitable as a scaffold for tissue engineering.

Thus, there remains a significant need for GTR and GBR barrier membranes for use in dental applications that combine the advantages of currently available synthetic materials with respect to stability and mechanical properties and the advantages of natural materials with respect to biocompatibility, thereby overcoming the structural, mechanical, and bio-functional limitations of currently available barrier membranes.

### SUMMARY

The present disclosure describes membranes suitable for use as guided tissue regeneration (GTR) barrier membranes and guided bone regeneration (GBR) barrier membranes in dental applications that are composed of fibrous and highly porous biodegradable materials fabricated using electrospinning and that may be surface-modified with plasma treatment or other suitable methods of surface-modification.

Specifically, there is provided a membrane for use in guided tissue regeneration and guided bone regeneration applications comprising one or more layers comprising a polymer comprising an amino acid-based poly(ester urea),
wherein the one or more layers are generated by one or more steps of electrospinning the polymer into electrospun polymer fibers.

The disclosed membranes have a high surface area to volume ratio. The use of the disclosed GTR barrier membranes or GBR barrier membranes provides a barrier that prevents the migration of soft tissue cells but is permeable to small molecules such as nutritional substances and medications. Methods of fabricating the disclosed resorbable barrier dental membranes for GTR and GBR applications using electrospinning are also disclosed. Electrospinning allows precise control over the pore size and microstructure characteristics of the membranes generated. The disclosed membranes may have precisely tuned physical, chemical, and mechanical properties optimized for various GTR and GBR applications.

### BRIEF DESCRIPTION OF THE DRAWINGS

Figure 1A shows representative SEM micrographs of a bilayer membrane.
Figure 1B shows fiber diameter distribution for the bilayer membrane of Figure 1A.
Figure 1C shows porosity measurements for the bilayer membrane of Figure 1A.
Figure 1D shows pore size results for the bilayer membrane of Figure 1A.
Figure 2 shows the results of wetting analysis testing via a DCA wicking experiment, showing the difference in total normalized weight gain during immersion between the pre-treated and post-treated samples.
Figure 3 shows representative tensile testing results of pre- and post-treated samples, including the stress-strain curve in Figure 3A, the load at break in Figure 3B, the tensile strain at break in Figure 3C, and the Young's modulus in Figure 3D.

### DETAILED DESCRIPTION OF THE ILLUSTRATED EMBODIMENTS

According to claim 1, the present disclosure describes membranes suitable for use as guided tissue regeneration (GTR) barrier membranes and guided bone regeneration (GBR) barrier membranes comprising two or more layers comprising a polymer comprising an amino acid-based poly(ester urea); wherein the two or more layers comprise a first layer and a second layer, wherein the first layer has a small average pore size and the second layer has a large average pore size, wherein the large average pore size is greater than the small average pore size wherein the two or more layers are generated by one or more steps of electrospinning a solution containing the polymer into electrospun polymer fibers; and wherein the membrane is surface-modified.

The membranes are suitable for use in dental applications that are composed of fibrous and highly porous biodegradable materials fabricated using electrospinning and that may be surface-modified with plasma treatment or other suitable methods of surface-modification. The use of the disclosed GTR barrier membranes or GBR barrier membranes provides a barrier that prevents the migration of soft tissue cells but is permeable to small molecules such as nutritional substances and medications. Permeability to small molecules results from the high surface area to volume ratio of the disclosed electrospun biodegradable materials.

As used herein, the term "membrane" refers to a thin, pliable sheetlike structure that may act as a boundary, lining, barrier, or partition. A membrane has two surfaces, which may be referred to as the top surface and the bottom surface, the inner surface and the outer surface, or according to other suitable designations of the surfaces. A membrane also has a thickness, corresponding to the orthogonal distance between the surfaces.

In some embodiments, a method of fabricating a resorbable barrier dental membrane for GTR and GBR applications using electrospinning is disclosed. Electrospinning allows precise control over the pore size and microstructure characteristics of the membranes generated using the disclosed methods. The GTR and GBR applications may include socket preservation, ridge augmentation, sinus lift, treating periodontal defects, and implant dehiscence.

In some embodiments, the disclosed methods may be used to generate GTR and GBR dental barrier membranes with precisely tuned physical properties. In some embodiments, the disclosed methods may be used to generate GTR and GBR dental barrier membranes with precisely tuned chemical properties. In some embodiments, the disclosed methods may be used to generate GTR and GBR dental barrier membranes with precisely tuned mechanical properties.

In some embodiments, the disclosed methods may be used to generate GTR and GBR dental barrier membranes with precisely tuned physical and chemical properties. In some embodiments, the disclosed methods may be used to generate GTR and GBR dental barrier membranes with precisely tuned physical and mechanical properties. In some embodiments, the disclosed methods may be used to generate GTR and GBR dental barrier membranes with precisely tuned chemical and mechanical properties.

In some embodiments, the disclosed methods may be used to generate GTR and GBR dental barrier membranes with precisely tuned physical, chemical, and mechanical properties.

The disclosed membranes may be fabricated using various synthetic or natural materials including amino acid-based poly(ester urea) (PEU), polydioxanone (PDO), polylactic-co-glycolic acid (PLGA), polylactic acid (PLA), polycaprolactone (PCL), 4-hydroxybutyrate (4HB), poly 4-hydroxybutyric acid (P4HB), chitosan, silk, or combinations thereof. In some embodiments, the amino acid-based poly(ester urea) may include one or more amino acids selected from the group consisting of L-leucine, L-isoleucine, L-valine, and L-phenylalanine. One example of a suitable PEU material is L-valine-co-L-phenylalanine poly(ester urea) (PEU) copolymer. This PEU material does not produce any acidic byproducts that would trigger inflammation when a membrane comprising said PEU material is used as a GTR barrier membrane or GBR barrier membrane.

Electrospinning may be performed using any known electrospinning setup suitable for electrospinning polymer fibers. In some embodiments, an electrospinning apparatus comprising a syringe pump, a syringe, a power supply, and a mandrel or drum for fiber collection is used to electrospin a polymer into electrospun polymer fibers to generate an electrospun construct. Electrospinning may be carried out in a single step or multiple steps. The electrospun construct may preferably be a membrane. In some embodiments, the polymer is dissolved in a solvent to generate a polymer solution, the polymer solution is added to the syringe, and the syringe is then loaded into the syringe pump prior to electrospinning of the polymer fibers.

In some embodiments, the solvent may be hexafluoroisopropanol (HFIP), dichloromethane, methanol, tetrahydrofuran (THF), acetone, chloroform, water, phosphate-buffered saline (PBS), or a combination thereof.

In some embodiments, the electrospun membrane generated using the disclosed methods may be composed of a single layer or multiple integrated layers. In some embodiments, the membrane generated may be composed of multiple integrated layers with distinguishable microstructure characteristics.

In some embodiments, the fiber diameter of the electrospun polymeric fibers may be between about 100 nm and about 15 µm.

In some embodiments, the thickness of the membrane may be between about 100 µm and 2 mm. In some preferred embodiments, the membrane may be composed of at least two layers with different pore sizes. This may preferably enhance the functionality of the membrane as a barrier for soft tissue that promotes healing. The layer with the smaller pore size may preferably function as a barrier during gingival tissue healing, preventing soft tissue infiltration into a bone defect and also stabilizing one or more blood clots formed during healing. The layer with the larger pore size may preferably promote cell infiltration and guided bone healing.

In some preferred embodiments, the membrane may be composed of three layers, where one layer has a small pore size and two layers have a large pore size and the layer that has a small pore size is situated between the two layers that have a large pore size. This configuration may enhance integration of the layer that has a small pore size within the membrane and may significantly reduce the risk of delamination. In some embodiments, the small pore size may be between about 1-20 µm and the large pore size may be between about 20-400 µm. In some embodiments, the pore size of a layer may be determined by adjusting the viscosity of the polymer solution and adjusting the electrospinning process conditions to stabilize the spinning jet. Solutions with lower viscosity may be used to produce layers having a small pore size, and solutions with higher viscosity may be used to produce layers having a large pore size.

In some embodiments, the mechanical integrity and binding forces between layers of the membrane may be enhanced by electrospraying short fibers prior to electrospinning the subsequent layer. In some other embodiments, the mechanical integrity and binding forces between layers of the membrane may be enhanced by electrospinning wet fibers by decreasing the screen distance to generate a "tacky surface" prior to electrospinning the subsequent layer.

**In** some preferred embodiments, a tubular braided structure or collapsible sleeve may be applied to the mandrel prior to electrospinning. The braid or sleeve may be metal or plastic. The braid or sleeve may facilitate the release of the electrospun construct from the mandrel. The use of a braid or sleeve may prevent damage to the morphology of the electrospun fibers during release from the mandrel.

**In** some preferred embodiments, residual solvent may be removed from the electrospun construct by heating the electrospun construct to a temperature below the glass transition temperature of the polymer in a convection oven or a vacuum oven. **In** some embodiments, residual solvent may be removed from the electrospun construct by immersing the electrospun construct in a solvent other than the residual solvent, whereby the residual solvent is removed from the electrospun construct via a liquid-liquid exchange mechanism. The solvent used to remove residual solvent may preferably be methanol. **In** some embodiments, residual solvent may be removed from the electrospun construct by heating the electrospun construct to a temperature below the glass transition temperature of the polymer in a convection oven or a vacuum oven and also separately immersing the electrospun construct in a solvent other than the residual solvent to remove the residual solvent via a liquid-liquid exchange mechanism. The residual solvent may preferably be removed to the extent that after the solvent removal the electrospun construct contains an amount of solvent that is less than physiologically acceptable tolerance limits for the solvent.

**In** some preferred embodiments, the surface chemistry of the electrospun membrane may be altered to enhance one or more properties including the wettability, conformability during use in dental surgery, and host tissue interactions of the membrane. The surface chemistry of the electrospun membrane may be altered using one or more methods selected from the group consisting of plasma treatment with one or more gases and blending with a non-ionic surfactant.

**In** some preferred embodiments, the gas used for plasma treatment may be introduced at a low pressure. **In** some embodiments, the plasma treatment may comprise treatment with at least one mixture of more than one gas. **In** some embodiments, the plasma treatment may comprise multiple separate and sequential treatment cycles comprising a first treatment cycle and a second treatment cycle, where the first treatment cycle comprises treatment with a first gas and the second treatment cycle comprises treatment with a second gas, where the first gas and second gas may be a single gas or a mixture of gases, and where the first gas differs in composition from the second gas. In some embodiments, the gas may be one or more gases selected from the group consisting of oxygen, nitrogen, argon, and ethylene oxide.

In some embodiments, the non-ionic surfactant may be pluronic-F108.

In some preferred embodiments, an additive or coating material may be added to the polymer solution or physically coated on the surface of electrospun membranes after electrospinning. The additive or coating material may be one or more additives or coating materials selected from the group consisting of platelet rich plasma (PRP), fibroblast growth factor (bFGF), hydroxyapatite, calcium phosphate, metronidazole (MNA), and *N-*methylpyrrolidone (NMP).

In some preferred embodiments, the surface of the electrospun membrane may be coated with an adhesive so that the membrane may be applied to a surgical site without suturing. The adhesive may be a biodegradable synthetic adhesive or a natural polymer. The adhesive may be one or more adhesives selected from the group consisting of poly(dopamine), fibrin glue, elastin, dihydroxyphenylalanine (DOPA) derivatives, polyethylene glycol (PEG), hyaluronic acid, polyethylene glycol (PEG) and its derivatives, alginate, calcium, gelatin, chitosan, polysaccharides, and poly amido amine (PAMAM) dendrimer.

In some preferred embodiments, an oxygen plasma treatment may be used to activate the surface of the electrospun membrane prior to coating with an adhesive. The activation of the surface of the electrospun membrane using oxygen plasma treatment will generate functional groups such as hydroxyls on the surface of the membrane to facilitate adhesion to the adhesive via a click chemistry mechanism.

In some embodiments, the electrospun membrane may be sized into a size that is suitable for use in dental applications using laser cutting and printing. In some embodiments, the membranes may be marked to identify a top side and a bottom side.

In some embodiments, the electrospun membrane may be sterilized using electron beam or gamma sterilization procedures.

In some preferred embodiments, the disclosed membranes possess excellent mechanical strength. This may facilitate suture retention in GTR barrier membrane and GBR barrier membrane applications. In addition, a mechanically sound membrane with sufficient load-bearing ability will be able to maintain a suitable physical space for the intended tissue or bone regeneration.

In some preferred embodiments, membranes generated using the disclosed methods are malleable. This may facilitate manipulation of the membranes to assume the specific geometry required to maximize functionality of the tissue or bone reconstruction in a specific application.

In some preferred embodiments, the disclosed membranes are fully resorbable when used in dental applications in humans. Resorbability is achieved via degradation of the membrane, and this obviates the need for a second surgery to remove a membrane used in a GTR or GBR application.

In some embodiments, the degradation rate of the membrane may be adjusted by adjusting the fiber diameter and thereby changing the total surface area to volume ratio, by adjusting the thickness of the membrane, or by adjusting both the fiber diameter and the thickness of the membrane.

In some preferred embodiments, the surface erosion and degradation mechanisms for the disclosed membranes are sufficiently predictable to allow resorption of the membranes within a specified time range under physiological conditions when used in GTR or GBR applications in humans. In some embodiments, the membrane resorbs in an amount of time between 45 and 75 days under physiological conditions. In some embodiments, the membrane resorbs in an amount of time between 105 and 135 days under physiological conditions. In some embodiments, the membrane resorbs in an amount of time between 165 and 195 days under physiological conditions.

In some embodiments, use of the disclosed electrospun GTR barrier membranes or GBR barrier membranes may facilitate osseointegration of dental implants placed with trans-mucosal healing elements immediately into tooth extraction sites. The GTR barrier membrane or GBR barrier membrane may preferably comprise an absorbable circumferential membrane arranged to exclude epithelial cells but not osteoblasts from the tooth extraction socket in which a dental implant is placed. As a result of this arrangement, the dental implant osseointegrates into the jaw of the patient without interruption from epithelial cells. Further, the soft texture of the electrospun fibers minimizes tissue irritation and therefore minimizes potential inflammation. These properties of fibrous electrospun scaffolds provide significant advantages over other GTR and GBR scaffolds for use in periodontal GTR and GBR barrier membrane applications.

The optimal degradation rate for GTR barrier membranes and GBR barrier membranes may be between four weeks and six months, depending on the clinical application in which the GTR barrier membranes or GBR barrier membranes are used. Increasing surface hydrophilicity and controlling the degradation rate of electrospun biodegradable materials is thus highly desirable for GTR and GBR barrier membrane applications. In some embodiments, plasma treatment of electrospun biodegradable materials may be used to introduce polar functional groups on the surface of the materials, thereby increasing the hydrophilicity of the surface. In some embodiments, the surface of the electrospun biodegradable material is first exposed to a gas at low pressure and then electrically stimulated to ignite the gas, thereby altering the surface chemistry of the material.

In some embodiments, growth factors may be incorporated into the disclosed electrospun biodegradable materials. The incorporation of growth factors into electrospun matrices for tissue engineering may enhance bioactivity by supplying appropriate physical and chemical cues to promote cellular proliferation and migration, thereby increasing the cellularization of the structures. The electrospun GTR barrier membrane or GBR barrier membrane may replicate the role of the native ECM in normal wound healing by serving as a reservoir of soluble growth factors critical to regeneration and providing a template for tissue repair. This may accelerate cellularization and tissue repair.

In some embodiments, platelet-rich plasma (PRP) therapy may be incorporated with electrospun polymeric GTR barrier membranes or polymeric GBR barrier membranes to harness the reparative potential and bioactivity found in a platelet-rich plasma. PRP therapy is a method of collecting and concentrating autologous platelets, through centrifugation and isolation, for the purpose of activating and releasing their dense, growth factor-rich granules. The discharge of these concentrated granules releases a number of growth factors and cytokines in physiologically relevant ratios, albeit in concentrations several times higher than that of normal blood, that are critical to tissue regeneration and cellular recruitment. Clinically, PRP therapy has been used to stimulate tissue growth and regeneration in a number of different tissues, effectively accelerating the healing response in patients suffering from osteochondral defects. The combination of a PRP with an electrospun polymeric GTR barrier membrane or polymeric GBR barrier membrane scaffold may generate a product that will provide the advantages of using electrospun biodegradable materials as a barrier for the epithelial layer in periodontium and the enhanced healing and regeneration of bone tissues by the sustained release of the PRP component.

A method of regenerating bone or tissue in a patient, wherein the method comprises applying a membrane selected from the group consisting of the membranes described herein into the patient's oral cavity, is also disclosed herein.

The following example is provided as a specific illustration of the disclosed methods and products. It should be understood, however, that the invention is not limited to the specific details set forth in the example.

Further, any range of numbers recited above or in the paragraphs hereinafter describing or claiming various aspects of the invention, such as ranges that represent a particular set of properties, units of measure, conditions, physical states, or percentages, is intended to literally incorporate any number falling within such range, including any subset of numbers or ranges subsumed within any range so recited. The term "about" when used as a modifier is intended to convey that the numbers and ranges disclosed herein may be flexible as understood by ordinarily skilled artisans and that practice of the disclosed invention by ordinarily skilled artisans using properties that are outside of a literal range will achieve the desired result.

### Materials and Methods

Materials: Amino acid-based poly(ester urea) poly(1-PHE-6) with a molecular weight of 45 kDa (hereinafter "PEU") was provided by Prof. Matthew Becker at the University of Akron. Hexafluoroisopropanol (HFIP) was purchased from Oakwood Products Inc., Estill, SC.

Solution Preparation: PEU was added to HFIP to generate 7% and 15% wt/vol solutions. The solutions were mixed on a stirring plate until the polymer pellets completely dissolved.

Barrier Membrane Fabrication: A bilayer membrane was produced via an electrospinning process as described. The prepared PEU solution was added to a syringe and loaded in a syringe pump connected to an electrospinning machine. The electrospinning setup included a programmable syringe pump (Model R99-E, Razel Scientific Instruments) attached to a glass syringe with a flat-end needle connected to a positive terminal of a high voltage power supply (0-30 kV) (EN 61010-1, Glassman High Voltage). The fibers were collected on a rotating aluminum mandrel with a 25 mm diameter at a rotation speed of 900 rpm.

A bilayer membrane was produced using the following procedure. First, the 15% solution was electrospun at a flow rate of 8 mL/h and an applied voltage of 13 kV. An 18 gauge needle was used, and the distance between the tip of the needle and the rotating mandrel was set to 20 cm. Second, the 7% solution was electrospun at a flow rate of 3 mL/h and an applied voltage of 16 kV. A 21 gauge needle was used, and the distance between the tip of the needle and the rotating mandrel was set to 25 cm. A total of 4 mL of the polymer solution was dispensed for each layer. The time between spinning processes between the two layers was less than ten minutes. The two layers were also electrospun separately for the purpose of characterizing the individual layers.

Post-Fabrication Treatment: The electrospun tube generated was dried on the mandrel in a vacuum oven at 35 degrees Celsius for 20 hours to remove residual solvent. After drying, the membrane was released off the mandrel and the electrospun tube was cut into a flat sheet. The sheet was then exposed to oxygen plasma surface treatment using a low-pressure plasma system (Diener FEMTO plasma system, Model FEMTO40KHZ) at a flow rate of 70% and applied energy of 30%. The electrospun sheet was then cut into multiple membranes of 20x30 mm using scissors. The membranes were then sterilized via E-beam at 30 kGy (Steri-Tek, Fremont, CA).

### Analyses

Morphology Analysis: The morphology of the electrospun membranes were analyzed by scanning electron microscopy (Zeiss, SUPRA 55VP). Membrane samples were sputter coated with platinum and palladium using a sputter coater for two minutes (Quorum Technologies, EMS 300T Dual Head) under a pressure of 8^{x}10⁻² mbar and an electric potential of 300 V.

Fiber Diameter Analysis: Fiber diameters were measured from SEM images using analysis software (FibraQuant 1.3.153, NanoScaffold Technologies, Chapel Hill, NC). At least 250 measurements were recorded on each scaffold type using top view SEM images of 2000x. These measurements were reviewed by an operator to confirm program accuracy.

Porosity Analysis: The porosity of the membranes was evaluated using a gravimetric measurement method. Using this method, porosity (*ε*) is defined in terms of the apparent density of the fiber mat (*ρ_{APP}*) and bulk density of the polymer (*ρₘₐₜₑᵣᵢₐₗ*) of which it is made: $ε = 1 - {ρ}_{APP} / {ρ}_{material}$ The apparent scaffold density *ρ_{APP}* was measured as a mass to volume ratio on 10 mm dry disks: ${ρ}_{APP} = mass / {V}_{material}$

Pore Size Analysis: The pore size of the membranes was estimated indirectly through approximate statistical models. *See* Kim, C.H., et al. J. Biomed. Mater. Res. Part B Appl. Biomater. 2006, 78B, 283; Eichhorn, S.J., et al. "Statistical Geometry of Pores and Statistics of Porous Nanofibrous Assemblies," J. R. Soc. Interface, 2005, 2, 309-18. The model yields the following approximated distribution *p(r)* of 3D pore radii *r* associated with a unimodal fiber distribution: $p \left(r\right) = \frac{n}{ε} {\left(\frac{Γ \left(k, bn\right)}{Γ \left(k\right)}\right)}^{n / ε - 1} \frac{{b}^{k}}{Γ \left(k\right)} {r}^{k - 1} exp \left(-br\right)$ where Γ(*k,bn*) and Γ(*k*) are the incomplete and complete gamma functions respectively, *k* is a constant parameter equal to 1.6, *n* is an equivalent number of layers, and *b* is an experimental parameter.

The experimental parameter is defined as *b =* 2*k* /〈*r_{2D}*〉, a function of the average bidimensional pore diameter 〈*r*_{2*D*}〉 of one fiber layer, which in turn is related to *ε* and to the average 〈*ω*〉 by $\left〈{r}_{2 D}\right〉 ≅ \frac{\sqrt{π}}{4} \left(\frac{π}{2 ln \left(1/ε\right)}-1\right) \left〈ω\right〉$ The distribution *p(r)* is conceived as the superposition of 2D layers, the number *n* of which was assumed to be: $n \left(ε, c\right) ≅ \frac{c}{ln \left(1/ε\right)}$ where the coverage parameter c is defined as: $c \left(β, \left〈ω\right〉\right) = \frac{total apparent scaffold volume}{volume of 1 - monolayer of fibers} ≅ \frac{4}{{πρ}_{PEU}} \frac{β}{\left〈ω\right〉}$ The coverage parameter corresponds to the average surface density, namely the ratio of the mass of the 20 mm disks and their surface area. Hence, the distribution p(r) is determined by inputting the set {〈ω〉,*ε,β*} of three experimentally-determined input parameters, and the average pore radius 〈*r*〉, taken as the representative measure for the scaffold, is simply $\left〈r\right〉 = {\int }_{0}^{∞} rp \left(r\right) dr$ A second model, *see* Sampson, W.W. "Modeling Stochastic Fibrous Materials with Mathematica," In Engineering Materials and Processes, XII, Berlin: Springer, 2009, was also employed to obtain a refined estimate for 〈*r*〉*.* The second model differs from the former only with respect to the definition of 〈*e*_{2*D*}〉: $\left〈{r}_{2 D}\right〉 ≅ \frac{\left〈ω\right〉}{ln \left(1/ε\right)}$ Both models were implemented in MAPLE (Maplesoft, Ontario, Canada) for symbolic computation.

Wettability Analysis: Wetting experiments were performed using a Cahn model 315 Dynamic Contact Angle (DCA) analyzer with WinDCA 32 v. 2.11 software. A sample was lowered into a test fluid, partially immersed in the fluid, and then withdrawn from the test fluid. The instrument records the changes in mass sensed by the balance as the process plots the changes as a function of sample position. The DCA wicking experiments were conducted using 10 mm wide specimens of the membrane samples. The instrument was programmed at a speed of 80 µm/s.

Mechanical Analysis: Tensile testing was performed on 10 mm x 50 mm samples that were mounted on an electromechanical load frame (Shimadzu AGS-X electromechanical load frame) using a 1 kN load cell. The testing parameters were the same for all samples, using a data acquisition rate of 100 Hz, a gauge length of 30 mm, and a test speed of 1 mm/s.

### Results

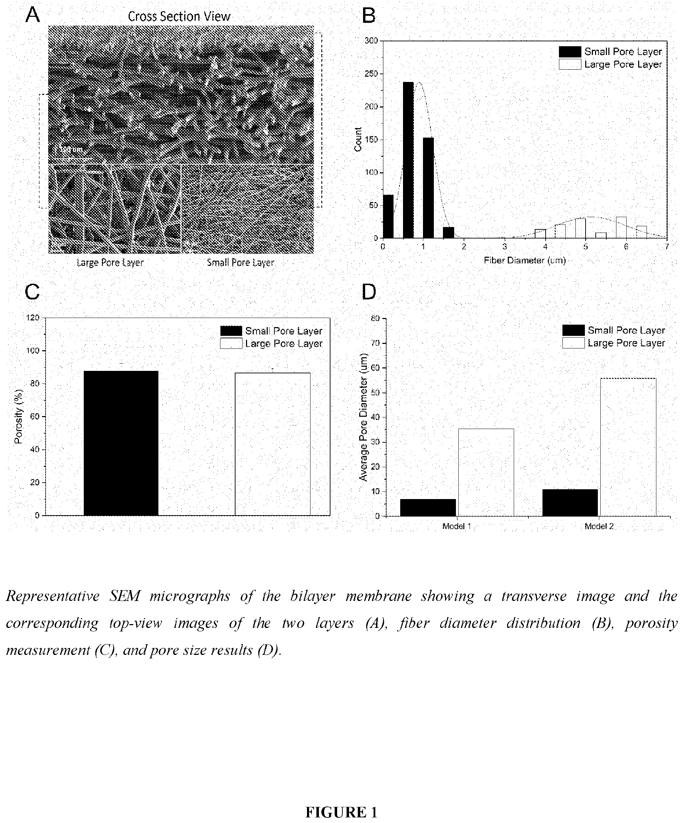

Morphology and Microstructure Characteristics: Representative SEM micrographs of cross-section and top-views of a representative membrane sample are shown in Figure 1A. In the cross-section, the fibers comprising each layer were discernable. The structure of the exterior side of the membrane (small pore layer) was composed of a smaller fiber diameter than the interior side of the membrane (large pore layer). The top-view images of both exterior and interior sides show that fibers were smooth and randomly oriented while no beads were observed. The thickness of the individual layers was about 50 µm and 300 µm for the small pore layer and large pore layer respectively. Based on the FibraQuant analysis, the average fiber diameter for the individual layers was 0.89 µm and 5.12 µm for the small pore layer and large pore layer respectively. The fiber diameter distribution is shown in Figure 1B. Based on the gravimetric measurements, the average porosity was 87.9% and 86.6% for the small pore layer and large pore layer respectively, as shown in Figure 1C. The average pore diameter for the individual layers was calculated by mathematical models. The models demonstrated that the layer constructed using fibers having a small fiber diameter had narrower pores than the layer constructed using fibers having a large fiber diameter. The average pore size for the small pore layer was 6.9 µm and 10.9 µm based on the first and second mathematical models described above, respectively. The average pore size for the large pore layer was 35.3 µm and 55.8 µm based on the first and second mathematical models described above, respectively.

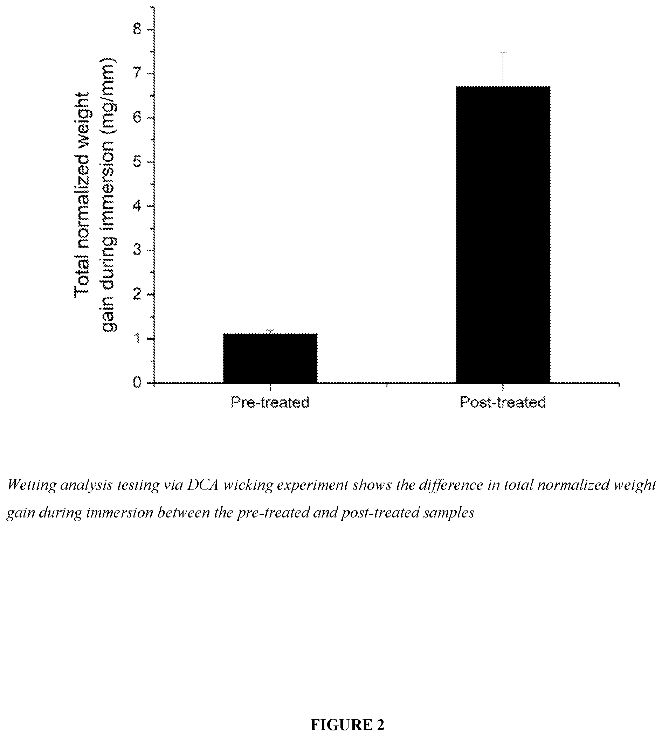

Wettability Characteristics: The effect of plasma surface treatment on the membrane's hydrophilicity was evaluated via a DCA wicking experiment. Both pre-treated and post-treated samples were tested. The total normalized weight gain during samples immersion was 1.1 mg/mm for the pre-treated sample and 6.8 mg/mm for the post-treated sample, as shown in Figure 2.

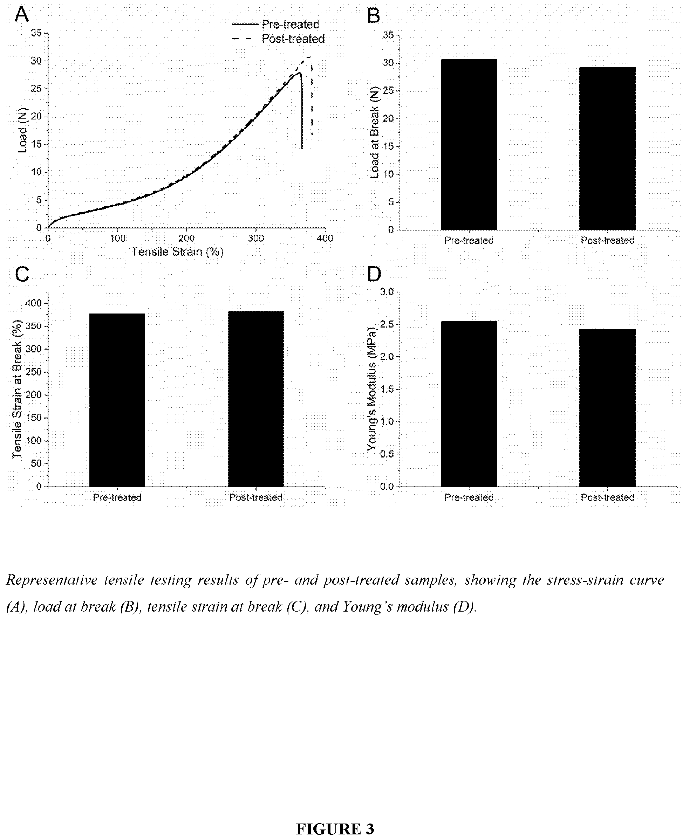

Mechanical Characteristics: The tensile strength of the membrane samples was evaluated. The tensile properties of both pre-plasma and post-plasma samples were almost identical, as shown in Figure 3A. Membrane samples had an ultimate tensile strength of 30 N as shown in Figure 3B, a tensile strain at break above 350% as shown in Figure 3C, and a Young's modulus of about 2.5 MPa as shown in Figure 3D.

### Discussion

In electrospun constructs, pore size is not an independent design parameter, but it is dependent on other microstructure characteristics. Among these microstructure characteristics, fiber diameter has the most significant impact on pore size. Thus, to generate a multi-layer membrane with different pore sizes for each of the layers, it is necessary to generate fibers with different diameters. Adjusting the viscosity of the solutions used for electrospinning allowed the generation of fibers with different diameters. In addition, adjustments to process parameters such as applied voltage, needle gauge size, and screen distance generated better results.

The electrospun fibers generated were smooth, without bead formation or other morphological defects. Moreover, the differences between small pore size and large pore size layers of the membranes generated were readily distinguishable by SEM. The fiber diameter of the large pore layer was significantly larger than the small pore layer, and the fiber diameter measurements exhibited narrow size distribution for both layers. This indicates that the spinning jet used in electrospinning was stable.

The non-woven structure produced by electrospinning usually features high surface area to volume ratio regardless of fiber diameter. Consistent with this expected result, the average porosity shown in Figure 1C did not show significant differences between the large pore size and small pore size layers.

Wettability testing demonstrated that the hydrophilicity of the membranes improved drastically after plasma treatment. Membrane hydrophilicity is critical for use as a dental barrier membrane, as this enhances conformability during placement of the membrane in a surgical site and facilitates handling of the membrane during surgery.

Mechanical testing showing that the tensile properties of the bilayer membrane were far superior to those of currently available GTR barrier membranes. Most importantly, undesirable delamination was not observed even at high strain conditions. This indicates that the two layers of the membrane were fully integrated and cannot be separated even under high tensile stress conditions.

The previous description of the disclosed embodiments is provided to enable any person skilled in the art to make or use the invention disclosed herein.

## Claims

1. A membrane for use in guided tissue regeneration and guided bone regeneration applications comprising two or more layers comprising a polymer comprising an amino acid-based poly(ester urea);
wherein the two or more layers comprise a first layer and a second layer, wherein the first layer has a small average pore size and the second layer has a large average pore size, wherein the large average pore size is greater than the small average pore size, determined according to SEM and methods in the description,
wherein the two or more layers are generated by one or more steps of electrospinning a solution containing the polymer into electrospun polymer fibers; and wherein the membrane is surface-modified.

2. The membrane for use according to Claim 1, wherein the diameters of the fibers in the first layer are smaller than the diameters of the fibers in the second layer.

3. The membrane for use of Claim 1 or Claim 2, wherein the polymer is poly(1-PHE-6); the solution further comprises the solvent hexafluoroisopropanol; the porosities of the first layer and the second layer are not significantly different; and wherein the membrane is surface-modified by blending a non-ionic surfactant into the solution prior to electrospinning.

4. The membrane for use of any one of Claims 1 to 3, wherein the polymer or combination of polymers comprises poly(1-PHE-6); the solution comprises the solvent hexafluoroisopropanol; wherein the porosities of the first layer and the second layer are not significantly different; and wherein the membrane is surface-modified with plasma treatment after all of the electrospinning steps are completed.

5. The membrane for use of any one of Claims 1 to 4, wherein each layer is integrated with each adjacent layer.

6. The membrane for use of any one of Claims 1 to 5, wherein the membrane comprises three layers, wherein the average pore size of the first layer is the small average pore size and the average pore size of the second and third layers is the large average pore size and the first layer is situated between the second and third layers, and wherein each layer is integrated with each adjacent layer.

7. The membrane for use of any one of Claims 1 to 6, wherein the small average pore size is between about 1-20 µm and the large average pore size is between about 20-400 µm, determined according to SEM and methods in the description.

8. The membrane for use of any one of Claims 1 to 7, wherein mechanical integration and binding between the layers is enhanced by:
a) electrospraying short fibers before electrospinning the subsequent layer; or
b) electrospinning wet fibers by decreasing the screen distance before electrospinning the subsequent layer.

9. The membrane for use of any one of Claims 1 to 8, wherein:
the one or more steps of electrospinning the polymer are implemented using an electrospinning apparatus comprising a syringe pump, a syringe, a power supply, and a mandrel or drum;
a tubular braided structure or collapsible sleeve is applied to the mandrel or drum before electrospinning.

10. The membrane for use of Claim 4, wherein the plasma treatment comprises treatment with one or more gases, for example one or more gases at a low pressure.

11. The membrane for use of Claim 10, wherein the plasma treatment comprises multiple separate and sequential treatment cycles comprising a first treatment cycle and a second treatment cycle, wherein the first treatment cycle comprises treatment with a first gas and the second treatment cycle comprises treatment with a second gas , optionally wherein the first gas differs in composition from the second gas.

12. The membrane for use of any of Claims 1 to 11, wherein the polymer further comprises polydioxanone.

13. The membrane for use of any one of Claims 1 to 12, wherein an additive or coating material is added to the polymer solution or physically coated on at least one surface of the membrane after all of the electrospinning steps are completed and optionally wherein the additive or coating material is one or more additives or coating materials selected from the group consisting of platelet rich plasma (PRP), fibroblast growth factor (bFGF), hydroxyapatite, calcium phosphate, metronidazole (MNA), and A-methyl pyrrolidone (NMP).

14. The membrane for use of any one of Claims 1 to 13, wherein at least one surface of the membrane is coated with an adhesive, and the adhesive is optionally one or more adhesives selected from the group consisting of poly(dopamine), fibrin glue, elastin, dihydroxyphenylalanine (DOPA) derivatives, polyethylene glycol (PEG), hyaluronic acid, polyethylene glycol (PEG) and its derivatives, alginate, calcium, gelatin, chitosan, polysaccharides, and poly amido amine (PAMAM) dendrimer, and optionally wherein an oxygen plasma treatment is used to activate at least one surface of the membrane prior to coating with the adhesive.

15. The membrane for use of any one of Claims 1 to 14, wherein the membrane resorbs under physiological conditions.

16. The use of a membrane as described in any one of Claims 1 to 15 for regenerating bone or tissue in a patient by applying the membrane into the patient's oral cavity.

## Patentansprüche

1. Membran zur Verwendung in Anwendungen zur gesteuerten Geweberegeneration und zur gesteuerten Knochenregeneration, umfassend zwei oder mehr Schichten, die ein Polymer umfassen, das einen Poly(esterharnstoff) auf Aminosäurebasis umfasst;
wobei die zwei oder mehr Schichten eine erste Schicht und eine zweite Schicht umfassen, wobei die erste Schicht eine kleine durchschnittliche Porengröße aufweist und die zweite Schicht eine große durchschnittliche Porengröße aufweist, wobei die große durchschnittliche Porengröße größer als die kleine durchschnittliche Porengröße ist, bestimmt gemäß REM und Verfahren in der Beschreibung,
wobei die zwei oder mehr Schichten durch einen oder mehrere Schritte des Elektrospinnens einer das Polymer enthaltenden Lösung zu elektrogesponnenen Polymerfasern erzeugt werden; und wobei die Membran oberflächenmodifiziert ist.

2. Membran zur Verwendung nach Anspruch 1, wobei die Durchmesser der Fasern in der ersten Schicht kleiner sind als die Durchmesser der Fasern in der zweiten Schicht.

3. Membran zur Verwendung nach Anspruch 1 oder Anspruch 2, wobei das Polymer Poly(1-PHE-6) ist; die Lösung ferner das lösliche Hexafluorisopropanol umfasst; die Porositäten der ersten Schicht und der zweiten Schicht sich nicht wesentlich unterscheiden; und wobei die Membran durch Mischen eines nichtionischen Tensids in die Lösung vor dem Elektrospinnen oberflächenmodifiziert wird.

4. Membran zur Verwendung nach einem der Ansprüche 1 bis 3, wobei das Polymer oder die Kombination von Polymeren Poly(1-PHE-6) umfasst; die Lösung das Lösungsmittel Hexafluorisopropanol umfasst; wobei sich die Porositäten der ersten Schicht und der zweiten Schicht nicht wesentlich unterscheiden; und wobei die Membran nach Abschluss aller Elektrospinnschritte durch Plasmabehandlung oberflächenmodifiziert wird.

5. Membran zur Verwendung nach einem der Ansprüche 1 bis 4, wobei jede Schicht mit jeder angrenzenden Schicht integriert ist.

6. Membran zur Verwendung nach einem der Ansprüche 1 bis 5, wobei die Membran drei Schichten umfasst, wobei die durchschnittliche Porengröße der ersten Schicht die kleine durchschnittliche Porengröße ist und die durchschnittliche Porengröße der zweiten und der dritten Schicht die große durchschnittliche Porengröße ist und sich die erste Schicht zwischen der zweiten und der dritten Schicht befindet, und wobei jede Schicht mit jeder angrenzenden Schicht integriert ist.

7. Membran zur Verwendung nach einem der Ansprüche 1 bis 6, wobei die kleine durchschnittliche Porengröße, bestimmt gemäß REM und Verfahren in der Beschreibung, zwischen etwa 1-20 µm und die große durchschnittliche Porengröße zwischen etwa 20-400 µm liegt.

8. Membran zur Verwendung nach einem der Ansprüche 1 bis 7, wobei die mechanische Integration und Bindung zwischen den Schichten verbessert wird durch:
a) Elektrospritzen kurzer Fasern vor dem Elektrospinnen der nachfolgenden Schicht; oder
b) Elektrospinnen nasser Fasern durch Verringern des Siebabstands vor dem Elektrospinnen der nachfolgenden Schicht.

9. Membran zur Verwendung nach einem der Ansprüche 1 bis 8, wobei:
der eine oder die mehreren Schritte des Elektrospinnens des Polymers unter Verwendung einer Elektrospinneinrichtung umgesetzt werden, die eine Spritzenpumpe, eine Spritze, eine Stromversorgung und einen Dorn oder eine Trommel umfasst;
eine rohrförmige geflochtene Struktur oder eine zusammenklappbare Hülse vor dem Elektrospinnen auf den Dorn oder die Trommel aufgebracht wird.

10. Membran zur Verwendung nach Anspruch 4, wobei die Plasmabehandlung eine Behandlung mit einem oder mehreren Gasen, zum Beispiel einem oder mehreren Gasen bei niedrigem Druck, umfasst.

11. Membran zur Verwendung nach Anspruch 10, wobei die Plasmabehandlung mehrere getrennte und aufeinanderfolgende Behandlungszyklen umfasst, die einen ersten Behandlungszyklus und einen zweiten Behandlungszyklus umfassen, wobei der erste Behandlungszyklus eine Behandlung mit einem ersten Gas umfasst und der zweite Behandlungszyklus eine Behandlung mit einem zweiten Gas umfasst, wobei sich optional das erste Gas in der Zusammensetzung von dem zweiten Gas unterscheidet.

12. Membran zur Verwendung nach einem der Ansprüche 1 bis 11, wobei das Polymer ferner Polydioxanon umfasst.

13. Membran zur Verwendung nach einem der Ansprüche 1 bis 12, wobei ein Additiv oder Beschichtungsmaterial nach Abschluss aller Elektrospinnschritte zu der Polymerlösung hinzugefügt oder auf mindestens einer Oberfläche der Membran physikalisch aufgetragen wird und wobei optional das Additiv oder Beschichtungsmaterial ein oder mehrere Additive oder Beschichtungsmaterialien ist, ausgewählt aus der Gruppe bestehend aus plättchenreichem Plasma (PRP), Fibroblastenwachstumsfaktor (bFGF), Hydroxylapatit, Calciumphosphat, Metronidazol (MNA) und A-Methylpyrrolidon (NMP).

14. Membran zur Verwendung nach einem der Ansprüche 1 bis 13, wobei mindestens eine Oberfläche der Membran mit einem Klebstoff beschichtet ist und der Klebstoff optional ein oder mehrere Klebstoffe ist, ausgewählt aus der Gruppe bestehend aus Poly(dopamin), Fibrinkleber, Elastin, Dihydroxyphenylalanin(DOPA)-Derivaten, Polyethylenglycol (PEG), Hyaluronsäure, Polyethylenglycol (PEG) und seinen Derivaten, Alginat, Calcium, Gelatine, Chitosan, Polysacchariden und Polyamidamin(PAMAM)-Dendrimer, und wobei optional eine Sauerstoffplasmabehandlung verwendet wird, um mindestens eine Oberfläche der Membran vor dem Beschichten mit dem Klebstoff zu aktivieren.

15. Membran zur Verwendung nach einem der Ansprüche 1 bis 14, wobei die Membran unter physiologischen Bedingungen resorbiert.

16. Verwendung einer Membran nach einem der Ansprüche 1 bis 15 zum Regenerieren von Knochen oder Gewebe bei einem Patienten durch Aufbringen der Membran in die Mundhöhle des Patienten.

## Revendications

1. Membrane destinée à être utilisée dans des applications de régénération tissulaire guidée et de régénération osseuse guidée comprenant deux couches ou plus comprenant un polymère comprenant une poly(ester urée) à base d'acides aminés ;
lesdites deux couches ou plus comprenant une première couche et une deuxième couche, ladite première couche présentant une petite taille de pore moyenne et ladite deuxième couche présentant une grande taille de pore moyenne, ladite grande taille de pore moyenne étant supérieure à la petite taille de pore moyenne, déterminée conformément à la MEB et aux procédés figurant dans la description,
lesdites deux couches ou plus étant générées par une ou plusieurs étapes d'électrofilage d'une solution contenant le polymère en fibres polymères électrofilées ; et ladite membrane étant modifiée en surface.

2. Membrane destinée à être utilisée selon la revendication 1, lesdits diamètres des fibres dans la première couche étant plus petits que les diamètres des fibres dans la deuxième couche.

3. Membrane destinée à être utilisée selon la revendication 1 ou la revendication 2, ledit polymère étant le poly(1-PHE-6) ; ladite solution comprenant en outre le solvant hexafluoroisopropanol ; lesdites porosités de la première couche et de la deuxième couche n'étant pas significativement différentes ; et ladite membrane étant modifiée en surface en mélangeant un tensioactif non ionique dans la solution avant l'électrofilage.

4. Membrane destinée à être utilisée selon l'une quelconque des revendications 1 à 3, ledit polymère ou ladite combinaison de polymères comprenant du poly(1-PHE-6) ; ladite solution comprenant le solvant hexafluoroisopropanol ; lesdites porosités de la première couche et de la deuxième couche n'étant pas significativement différentes ; et ladite membrane étant modifiée en surface par un traitement par plasma une fois que toutes les étapes d'électrofilage sont terminées.

5. Membrane destinée à être utilisée selon l'une quelconque des revendications 1 à 4, chaque couche étant intégrée à chaque couche adjacente.

6. Membrane destinée à être utilisée selon l'une quelconque des revendications 1 à 5, ladite membrane comprenant trois couches, ladite taille de pore moyenne de la première couche étant la petite taille de pore moyenne et ladite taille de pore moyenne des deuxième et troisième couches étant la grande taille de pore moyenne et ladite première couche étant située entre les deuxième et troisième couches, et chaque couche étant intégrée à chaque couche adjacente.

7. Membrane destinée à être utilisée selon l'une quelconque des revendications 1 à 6, ladite petite taille de pore moyenne étant comprise entre environ 1 et 20 µm et ladite grande taille de pore moyenne étant comprise entre environ 20 et 400 µm, déterminée conformément à la MEB et aux procédés figurant dans la description.

8. Membrane destinée à être utilisée selon l'une quelconque des revendications 1 à 7, l'intégration mécanique et la liaison entre les couches étant améliorées par :
a) électropulvérisation de fibres courtes avant l'électropulvérisation de la couche suivante ; ou
b) électrofilage de fibres humides en diminuant la distance du tamis avant l'électrofilage de la couche suivante.

9. Membrane destinée à être utilisée selon l'une quelconque des revendications 1 à 8 :
lesdites une ou plusieurs étapes d'électrofilage du polymère étant mises en œuvre à l'aide d'un appareil d'électrofilage comprenant une pompe à seringue, une seringue, une alimentation électrique et un mandrin ou un tambour ;
une structure tressée tubulaire ou un manchon pliable étant appliqué sur le mandrin ou le tambour avant l'électrofilage.

10. Membrane destinée à être utilisée selon la revendication 4, ledit traitement par plasma comprenant un traitement avec un ou plusieurs gaz, par exemple un ou plusieurs gaz à basse pression.

11. Membrane destinée à être utilisée selon la revendication 10, ledit traitement par plasma comprenant de multiples cycles de traitement distincts et séquentiels comprenant un premier cycle de traitement et un second cycle de traitement, ledit premier cycle de traitement comprenant un traitement avec un premier gaz et ledit second cycle de traitement comprenant un traitement avec un second gaz, ledit premier gaz différant éventuellement en composition du second gaz.

12. Membrane destinée à être utilisée selon l'une quelconque des revendications 1 à 11, ledit polymère comprenant en outre de la polydioxanone.

13. Membrane destinée à être utilisée selon l'une quelconque des revendications 1 à 12, un additif ou un matériau de revêtement étant ajouté à la solution de polymère ou appliqué physiquement sur au moins une surface de la membrane une fois que toutes les étapes d'électrofilage sont terminées et éventuellement ledit additif ou ledit matériau de revêtement étant un ou plusieurs additifs ou matériaux de revêtement choisis dans le groupe constitué par le plasma riche en plaquettes (PRP), le facteur de croissance des fibroblastes (bFGF), l'hydroxyapatite, le phosphate de calcium, le métronidazole (MNA) et l'A-méthyl pyrrolidone (NMP).

14. Membrane destinée à être utilisée selon l'une quelconque des revendications 1 à 13, au moins une surface de la membrane étant revêtue d'un adhésif, et ledit adhésif étant éventuellement un ou plusieurs adhésifs choisis dans le groupe constitué par la polydopamine, la colle de fibrine, l'élastine, les dérivés de dihydroxyphénylalanine (DOPA), le polyéthylène glycol (PEG), l'acide hyaluronique, le polyéthylène glycol (PEG) et ses dérivés, l'alginate, le calcium, la gélatine, le chitosane, les polysaccharides et un dendrimère de polyamidoamine (PAMAM), et éventuellement un traitement par plasma d'oxygène étant utilisé pour activer au moins une surface de la membrane avant le revêtement avec l'adhésif.

15. Membrane destinée à être utilisée selon l'une quelconque des revendications 1 à 14, ladite membrane se résorbant dans des conditions physiologiques.

16. Utilisation d'une membrane comme décrite selon l'une quelconque des revendications 1 à 15 destinée à régénérer un os ou un tissu chez un patient en appliquant la membrane dans la cavité buccale du patient.
